# EUROPÄISCHE PATENTSCHRIFT

(11) **EP 0 646 380 B1**
(45) Veröffentlichungstag und Bekanntmachung des Hinweises auf die Patenterteilung: **25.06.1997**
(21) Anmeldenummer: 94113197.1
(22) Anmeldetag: 24.08.1994
(51) Int. Cl.: B01D 19/00, A61M 1/36

(54) **Luftabscheider**
Air separator
Séparateur d'air

(30) Priorität: 01.09.1993 DE 4329385
(43) Veröffentlichungstag der Anmeldung: 05.04.1995
(73) Patentinhaber: Fresenius AG, D-61350 Bad Homburg v.d.H (DE)
(72) Erfinder: Heilmann, Klaus, D-66606 St. Wendel (DE); Knierbein, Bernd, Dr., D-66606 St. Wendel (DE)
(74) Vertreter: Fuchs, Luderschmidt & Partner Patentanwälte

(56) Entgegenhaltungen:
- GB-A- 2 041 233
- GB-A- 2 063 108
- US-A- 4 368 118
- DATABASE WPI Week 8645, 20. November 1986 Derwent Publications Ltd., London, GB; AN 86-297435/45 & SU-A-1 219 112 (CHELYABINSK POLY) 23. März 1986

## Beschreibung

Die Erfindung betrifft einen Luftabscheider für Gase enthaltende, durch eine Leitung geführte Flüssigkeit, insbesondere zum Abscheiden von Luftblasen aus Blut, mit einer im wesentlichen kreiszylinderförmigen Kammer, die von der Flüssigkeit im wesentlichen auf schraubenlinienförmigen Strömungsbahnen durchflossen werden kann, so daß die Gasblasen wegen der durch die auftretenden Zentrifugalkräfte erzeugten Druckunterschiede in radialer Richtung zur Längsachse der Kammer gedrängt werden können. Ein solcher gattungsgemäßer Luftabscheider weist weiterhin einen Einlaufstutzen und einen Auslaufstutzen auf, mittels derer er mit einer Leitung, beispielsweise in Form eines Schlauches, verbunden werden kann.

Ein solcher Luftabscheider, der - insofern er auch für andere Gase als Luft verwendet wird - allgemeiner auch als Entgasungsvorrichtung bezeichnet wird, ist beispielsweise aus der offengelegten Britischen Patentanmeldung 2 063 108 bekannt. Der dort beschriebene Luftabscheider weist eine im wesentlichen kreiszylinderförmig ausgebildete, senkrecht angeordnete Kammer auf, an deren oberen Ende ein Einlaßstutzen so angeordnet ist, daß die zu entgasende Flüssigkeit im wesentlichen tangential im Bereich des äußeren Umfangs eintritt. Durch die tangentiale Einleitung fließt die zu entgasende Flüssigkeit zunächst auf einer kreisförmigen Strömungsbahn, die jedoch von der Gesamtströmung durch die senkrechte Kammer überlagert wird, so daß die Flüssigkeit die Kammer auf einer schraubenlinienförmigen Strömungsbahn durchfließt. Am unteren Ende ist entsprechend ein tangential angeordneter Ablaufstutzen vorgesehen, so daß die entgaste Flüssigkeit austreten kann. Der Entgasungseffekt wird bei dem gattungsgemäßen Luftabscheider bzw. der Entgasungsvorrichtung bewirkt, indem durch die kreisförmigen Bewegungsanteile der Flüssigkeitsströmung Zentrifugalkräfte erzeugt werden, die in der Flüssigkeit Druckunterschiede aufbauen, so daß die weniger dichten, d.h. leichteren Luftblasen zur Mitte der Kammer gedrängt werden und längs der Längsachse der Kammer aufsteigen, bis sie durch eine Entlüftungsbohrung abgeführt werden.

Der beschriebene Luftabscheider wird insbesondere zur Entgasung von Blut eingesetzt. Es ist immer dann notwendig Blut von eventuell enthaltenen Gasen zu trennen, wenn Blut dem natürlichen Blutkreislauf eines Patienten entnommen und durch einen künstlichen Blutkreislauf geleitet wird, bevor es wieder in den Körper des Patienten zurückgeleitet wird. Dies kommt beispielsweise bei der Zellseparation im Rahmen der Autotransfusion von Blut bei Operationen vor, weiter bei der Hämodialyse oder Hämofiltration, sowie bei Mischformen dieser Behandlungstechniken.

Insbesondere bei der Entgasung von Blut stellt sich das Problem, daß zwar einerseits die Abscheidung von enthaltenen Luftblasen mit großer Zuverlässigkeit erfolgen muß, da eventuell im Blut verbleibende Luftblasen zum Tode des Patienten führen können, andererseits aber der Luftabscheider hinsichtlich seiner mechanischen Eigenschaften und der ausgebildeten Strömungsform so beschaffen sein muß, daß Beschädigungen der Blutbestandteile vermieden werden. Für eine geringe Blutschädigung ist ein gutes Auswaschverhalten des Luftabscheiders wünschenswert, was einhergeht mit materialseitig glatten Oberflächen sowie mit einer strömungsgünstigen kontinuierlichen Gestaltung der Strömungsbahnen, so daß das Anhaften von Blutkörperchen an Oberflächen des Luftabscheiders und damit eine Konglomeration von Blutkörperchen vermieden wird. Für eine geringe Blutschädigung weiterhin förderlich sind kurze Verweilzeiten des Blutes im Luftabscheider, ohne jedoch die Luftabscheidung als solche zu verschlechtern, sowie ein kleines Füllvolumen.

Für im Krankenhausbetrieb eingesetzte Luftabscheider ist es darüber hinaus wünschenswert, daß sie sich leicht befestigen lassen und ohne großen Aufwand in bereits bestehende Schlauchleitungen eingefügt werden können. Aus diesem Grund ist es wünschenswert, daß Einlaß- und Auslaßstutzen koaxial miteinander fluchtend angeordnet sind, so daß der Luftabscheider beispielsweise nach Aufschneiden eines existierenden Schlauches in diesen eingesetzt werden kann, ohne daß die Schlauchführung geändert werden müßte. Weiterhin kann beispielsweise die Schlauchführung an Dialysemaschinen ohne unnötige Schlaufen erfolgen und das Herstellungsverfahren für eine aus Luftabscheider und Schlauch bestehende vorgefertigte Einheit wird vereinfacht, da die Schläuche automatisch montiert werden können.

In allen die Sicherheit eines Patienten betreffenden Systemen ist darüber hinaus eine ständige Kontrollmöglichkeit wünschenswert, so daß eine optische Überwachung des Füllstands möglich sein soll. Weiterhin sind kapazitive Füllstandsüberwachungen denkbar, für deren ordnungsgemäßes Funktionieren wiederum ein gutes Auswaschverhalten notwendig ist, um das Verbleiben von Restblut im Luftabscheider zu verhindern.

Der aus der genannten britischen Patentanmeldung bekannte Luftabscheider erfüllt die oben genannten Forderungen nur teilweise. Insbesondere weist er keine koaxiale, in der Längsrichtung der Kammer liegenden Anschlüsse bzw. Stutzen auf, so daß seine Handhabung umständlich ist und das Auswaschverhalten hohen Anforderungen nicht genügt.

Da andererseits die schraubenlinienförmige Strömungsausbildung eine gute Luftabscheidung gewährleistet, liegt der Erfindung zunächst die Aufgabe zugrunde, einen Luftabscheider zu schaffen, der die Vorteile der schraubenlinienförmigen Strömungsführung beibehält und trotzdem in der Längsachse des Luftabscheiders liegende, koaxial zueinander angeordnete Anschlüsse bzw. Stutzen ermöglicht.

Insbesondere bei der Verwendung eines gattungsgemäßen Luftabscheiders zur Behandlung von Blut ist es im Krankenhausbetrieb wünschenswert, wenn der Luftabscheider selbst als Wegwerfteil ausgebildet ist, d.h., wenn er nach einer gewissen Einsatzzeit und insbesondere natürlich einem Patientenwechsel ausgetauscht und entsorgt werden kann, da eine Reinigung, die den hygienischen Anforderungen des Krankenhausbetriebes Genüge täte, vom Aufwand her nicht zu vertreten wäre. Es ist daher weiterhin eine Aufgabe der vorliegenden Erfindung, die oben beschriebenen, zu erzielenden Vorteile mittels eines neu zu schaffenden Luftabscheiders so zu realisieren, daß der vorgeschlagene Luftabscheider aus üblichen Kunststoffen kostengünstig in großen Stückzahlen, insbesondere durch Spritzgießen, herstellbar ist.

Die Lösung der Aufgabe ist bei einem gattungsgemäßen Luftabscheider dadurch gekennzeichnet, daß Einlaufstutzen und Ablaufstutzen in der Längsachse der Kammer liegend angeordnet sind, und daß zum Erzeugen des schraubenlinienförmigen Strömungsbildes stromab des Einlaufstutzens ein Strömungsleitbauteil - im folgenden auch Einlaufverteiler genannt - angeordnet ist, das im wesentlichen aus einem rotationssymmetrischen Rundkörper besteht, dessen der einströmenden Flüssigkeit zugewandte äußere Oberfläche (auch als erste Leitfläche bezeichnet) geometrisch durch Rotation eines Kurvenabschnitts um die Längsachse der Kammer so definiert ist, daß die einströmende Flüssigkeit zunächst radial nach außen geleitet werden kann, und daß auf der Leitfläche Leitschaufeln angeordnet sind, die zur Erzeugung der kreisförmigen Bewegungsanteile der Strömung in auf der Längsachse der Kammer senkrecht stehenden Ebenen gekrümmt und so umgelenkt sind, daß die Flüssigkeit beim Austritt aus dem Einlaufverteiler im wesentlichen tangential zur kreisförmigen Wandung der Kammer strömen kann. Dabei ist der Kurvenabschnitt vorzugsweise ein Ellipsenabschnitt.

Durch die erfindungsgemäße Verwendung eines stromab des Einlaufstutzens angeordneten Strömungsleitbauteils (Einlaufverteilers) wird es ermöglicht, eine Anordnung von Einlaufstutzen und Ablaufstutzen in der Längsachse der Kammer vorzusehen, ohne daß auf die Vorteile des Grundkonzepts, die Strömung schraubenlinienförmig auszubilden und die auftretenden Zentrifugalkräfte zur Entgasung des Blutes zu nutzen, verzichtet werden muß. Durch die beschriebene Gestaltung des Einlaufverteilers wird einerseits sichergestellt, daß sich dieses Bauteil wegen seiner geometrischen Form leicht aus üblichen Kunststoffen, insbesondere durchsichtigen, im Spritzgießverfahren herstellen läßt, andererseits wird eine kontinuierliche, Stöße weitgehend vermeidende Strömungsführung ermöglicht, so daß im Blut oder der gegebenenfalls anderen zu entgasenden Flüssigkeit ein gleichmäßiges Strömungsprofil erzeugt wird, das mit niedrigen und gleichmäßigen Schubspannungen und demgemäß geringer Blutschädigung einhergeht.

Besonders vorteilhaft ist dabei eine Ausgestaltung des rotationssymmetrischen Grundkörpers des Einlaufsverteilers, bei dem die äußere Oberfläche bzw. die erste Leitfläche durch Rotation eines konkav gekrümmten Kurvenabschnitts um die Längsachse der Kammer definiert ist. Durch eine solche Ausbildung wird ein stoßfreier Einlauf des Blutes in den Luftabscheider begünstigt.

Andererseits kann vorgesehen sein, den rotationssymmetrischen Grundkörper des Einlaufverteilers kegelförmig zu gestalten. Eine solche Ausgestaltung des Einlaufverteilers wird zwar mit etwas höheren Stoßverlusten im Einlaufbereich erkauft, bietet jedoch neben fertigungstechnischen Vorteilen den weiteren Vorteil, daß im Innenraum des Kegels die Mündung eines Tauchrohres angeordnet sein kann, was die Möglichkeit eröffnet, den Luftabscheider für einen Durchfluß von unten nach oben auszulegen.

Vorteilhaft ist weiterhin, eine zweite Leitfläche parallel zur ersten angeordnet vorzusehen, wobei vorzugsweise die Höhe der Leitschaufeln dann so bemessen ist, daß sie sich senkrecht zur Strömungsrichtung von einer Leitfläche bis zur anderen erstrecken. Jeweils zwei Leitschaufeln und die beiden parallel zueinander angeordneten Leitflächen begrenzen dann jeweils einen Strömungskanal, wodurch die Strömung genauer geführt werden kann und Strömungsablösungen, die Induzierung von Wirbeln etc. verhindert werden kann. Eine solche Ansgestaltung trägt also dazu bei, Verwirbelungen mit den damit verbundenen hohen Schubspannungen zu vermeiden, so daß die Ausgestaltung insbesondere unter dem Gesichtspunkt einer möglichst minimalen Blutschädigung vorteilhaft ist.

Bei der bereits beschriebenen Anordnung des Luftabscheiders der Gestalt, daß er von unten nach oben durchflossen wird, kann vorteilhafterweise vorgesehen sein, daß die Außenwandung der Kammer bereichsweise kegelförmig ausgebildet ist und die Innenseite der Wandung gleichzeitig als zweite Leitfläche fungiert.

Im folgenden werden weitere Vorteile und Merkmale des erfindungsgemäßen Luftabscheiders anhand von in den Zeichnungen dargestellten Ausführungsbeispielen näher beschrieben.

In der Zeichnung zeigen:
- **Fig. 1**: einen Längsschnitt durch einen senkrecht angeordneten Luftabscheider gemäß einer ersten Ausführungsform der Erfindung, der für eine Durchflußrichtung von oben nach unten vorgesehen ist,
- **Fig. 2**: eine perspektivische Darstellung des Strömungsverteilers mit aufgesetzter zweiter Leitfläche,
- **Fig. 3**: eine perspektivische Ansicht des Einlaufverteilers aus Fig. 2 mit abgenommener zweiter Leitfläche,
- **Fig. 4**: eine Draufsicht des Einlaufverteilers gemäß Fig. 3, und
- **Fig. 5**: einen Längsschnitt durch einen Luftabscheider gemäß einer zweiten Ausführungsform der Erfindung, der für einen Durchfluß von unten nach oben ausgelegt ist.

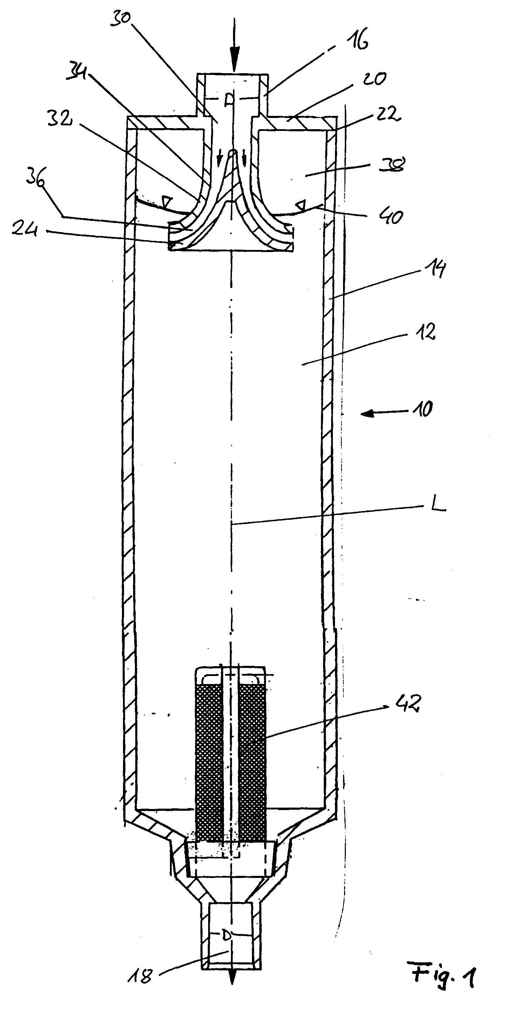

In Fig. 1 ist ein erfindungsgemäßer Luftabscheider 10 im Längsschnitt dargestellt, der im wesentlichen aus einer senkrecht angeordneten kreiszylinderförmigen Kammer 12 besteht, die von einer Wandung 14 begrenzt wird. Am oberen Ende der Kammer 12 ist ein Einlaufstutzen 16 und am unteren Ende ein Ablaufstutzen 18 angeordnet, die jeweils einen Innendurchmesser D aufweisen, der zusammen mit dem Durchmesser eines einzufügenden Schlauches eines Preßpassung bildet. Der Einlaßstutzen 16 ist einstückig mit einem Deckelbauteil 20 ausgebildet, das als Kunststoffteil im Spritzgießverfahren hergestellt ist und mit der ebenfalls im Spritzgießverfahren hergestellten Wandung 14 der Kammer 12 durch Verschweißen o.ä. an einer Nahtfüge 22 verbunden ist.

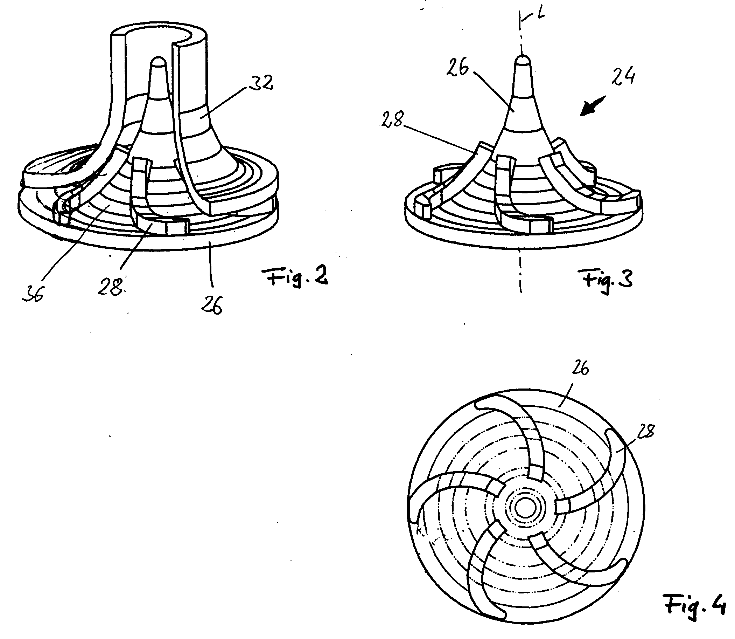

Stromabwärts des Einlaßstutzens 16 ist ein Einlaufverteiler 24 angeordnet, der allgemeiner im Rahmen dieser Beschreibung auch als Strömungsleitbauteil bezeichnet wird. Der Einlaufverteiler 24 ist in Fig. 3 perspektivisch dargestellt. Er besteht aus einem rotationssymmetrischen Grundkörper 26, dessen der einströmenden Flüssigkeit zugewandte äußere Oberfläche als erste Leitfläche bezeichnet wird. Diese erste Leitfläche ist geometrisch definiert durch Rotation eines konkaven Kurvenabschnitts um die Langsachse L der Kammer. Auf der ersten Leitfläche sind Leitschaufeln 28 angeordnet, die der konkaven Krümmung der Leitfläche des Rotationskörpers 26 folgen. Die Leitschaufeln sind zusätzlich, wie in Fig. 4 verdeutlicht, in Ebenen gekrümmt, die senkrecht auf der Längsachse L der Kammer stehen. Durch diese Ausgestaltung der Leitschaufeln 28 wird die in Richtung des oberen Pfeils in Fig. 1 in den Einlaufstutzen 16 einströmende Flüssigkeit durch die konkave Krümmung des Grundkörpers 26 zunächst radial nach außen geleitet und durch die Krümmung der Schaufeln 28 in auf der Längsachse L senkrecht stehenden Ebenen zusätzlich so umgelenkt, daß die Flüssigkeit beim Austritt aus dem Einlaufverteiler 24 im wesentlichen tangential zur kreisförmigen Wandung 14 der Kammer 12 strömt, wodurch die gewünschte schraubenlinienförmige Strömung induziert wird.

Wie Fig. 1 zeigt, ist zusätzlich zu dem Einlaufstutzen 16, der einstückig mit dem Deckelbauteil 20 ausgebildet ist, stromabwärts einer Einflußöffnung 30 ein weiterer Flansch 32 einstückig mit dem Deckelbauteil 20 ausgebildet, dessen innere Oberfläche 34 als zweite Leitfläche parallel zur ersten Leitfläche des Einlaufverteilers 24 angeordnet ist. Der Einlaufverteiler 24 ist ebenfalls im Spritzgießverfahren hergestellt und mit der zweiten Leitfläche 34 über die Oberseiten 36 der Leitschaufeln 28 verschweißt oder durch eine Klemmverbindung befestigt. Der Durchmesser der Einlaßöffnung 30 ist vorzugsweise so gewählt, daß er dem Innendurchmesser des in den Stutzen 16 einzuführenden Schlauches entspricht, so daß keine eine Strömungsablösung verursachenden Kanten verbleiben, sondern vielmehr ein glatter Übergang zwischen der Innenseite des zuführenden Schlauches und der zweiten Leitfläche 34 entsteht. Die zweite Leitfläche 34 begrenzt zusammen mit der ersten Leitfläche des Grundkörpers 24 einen im wesentlichen ringförmigen Spalt, der durch die Leitschaufeln 28 (vgl. Fig. 3) in Strömungskanäle unterteilt wird. Die Strömungskanäle 36 sind besonders deutlich in Fig. 2 zu sehen, das den Grundkörper 26, die Leitschaufeln 28 und den Flansch 32 zeigt. Der Flansch 32 ist dabei als unabhängiges Einzelteil gezeichnet, vorzugsweise ist jedoch vorgesehen, daß er, wie aus Fig. 1 ersichtlich, einstückig mit dem Deckelbauteil 20 ausgebildet ist.

Das einlaufende Blut oder die sonstige zu entgasende Flüssigkeit tritt durch den Einlaufstutzen 16 und fließt durch die Strömungsleitkanäle 36, wodurch die Strömungsrichtung in einer räumlich gewundenden Kurve aus der zunächst parallel zur Längsachse 11 verlaufenden Richtung in eine tangential an die Wandung 14 der Kammer 12 verlaufende Richtung umgelenkt wird. Hierdurch wird eine schraubenlinienförmige Strömung induziert, wobei die kreisförmigen Bewegungsanteile einen Druckunterschied aufbauen, der dazu führt, daß die Luftblasen in Richtung auf die Längsachse gedrängt werden und aufgrund ihrer geringeren Dichte nach oben aufsteigen. Die aufgestiegenen Luftblasen bilden im oberen Teil der Kammer 12 ein Luftpolster 38, das durch eine geeignete, in Fig. 1 nicht dargestellte Entlüftungsbohrung abgeleitet werden kann. Der Flüssigkeitsspiegel 40 des eingelaufenen Blutes ist aufgrund der Drehung leicht parabelförmig ausgebildet, wobei die in Fig. 1 dargestellte Parabel überhöht gezeichnet ist. Generell sind möglichst geringe Strömungsgeschwindigkeiten anzustreben, um eine erneute Zufuhr von Luft an der Oberfläche 40 der Flüssigkeit zu verhindern. Wie Fig. 1 weiter zeigt, ist der Auslauf des Einlaufverteilers 24 unterhalb des Flüssigkeitsspiegels 40 angeordnet.

Nachdem das Blut von oben nach unten in schraubenlinienförmigen Bahnen durch die Kammer 12 geflossen ist, fließt es durch eine zusätzlich vorgesehene Filterkerze 42 und dann durch den Auslauf 18 zur weiteren Verwendung ab.

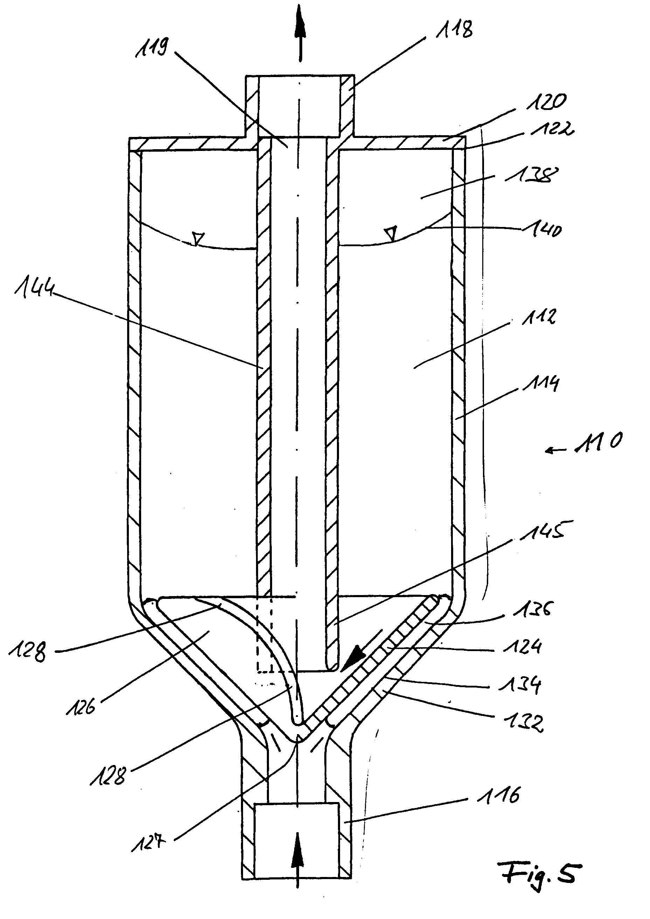

In Fig. 5 ist eine alternative Ausführungsform eines erfindungsgemäßen Luftabscheiders dargestellt, bei dem eine von einer Wandung 114 umgebene Kammer 112 von unten nach oben durchflossen wird. Bei dem in Fig. 5 dargestellten Ausführungsbespiel sind gleiche oder vergleichbare Teile mit den gleichen Bezugszeichen, erhöht um den Wert 100, bezeichnet. Der Einlaufverteiler 124 weist bei diesem Ausführungsbeispiel einen Grundkörper 126 auf, der im wesentlichen kegelförmig ist. Dies hat zur Folge, daß beim Auftreffen der durch den Einlaufstutzen 116 tretenden Flüssigkeit auf die Spitze 127 des Kegels wegen der diskontinuierlichen Umlenkung Stoßverluste in Kauf genommen werden müssen, jedoch bietet diese Ausführungsform neben einer für fertigungstechnische Zwecke günstigen Ausgestaltung den weiteren Vorteil, daß im Inneren des Kegels 126 Platz für die Mündung eines mit der Abflußöffnung 119 verbundenen Tauchrohres 144 verbleibt. Die durch den Einlaßstutzen 116 tretende Flüssigkeit fließt zunächst durch die Strömungsleitkanäle 136, die wie bei dem zuvor besprochenen Ausführungsbeispiel begrenzt durch eine erste Leitfläche werden, die die Oberfläche des kegelförmigen Grundkörpers 126 des Einlaufsverteilers 124 darstellt, sowie durch jeweils zwei Leitschaufeln 128 und eine zweite Führungsfläche 134, die beim vorliegenden Ausführungsbeispiel durch einen Wandungsteil 132 der die Kammer bildenden Wandung 114 gebildet ist. Nach dem Austritt aus dem Einlaufverteiler 124 steigt die Strömung in schraubenlinienförmigen Stromlinien nach oben auf, wobei enthaltene Luftblasen durch die aufgrund von Zentrifugalkräften erzeugten Druckunterschiede nach innen gedrängt werden und sich in einem Luftpolster 138 sammeln, das - wie bereits zuvor beschrieben - durch eine nicht dargestellte Entlüftungsbohrung im Deckelbauteil 120 abgebaut werden kann.

Aufgrund des aufgebauten Drucks wird Flüssigkeit durch die Mündung 145 des Tauchrohres 144 gedrückt und steigt in diesem auf, um durch den Ablaufstutzen 118 abzufließen. Vorteilhaft ist dabei, daß es durch das tiefreichende Tauchrohr 144 ermöglicht wird, Blut aus den strömungsberuhigten Gebieten der Kammer 112 abzuziehen, was die Entgasung weiter vorteilhaft unterstützt. Das Tauchrohr 144 ist vorteilhafterweise zusammen mit dem Ablautstutzen 118 einstückig mit dem Deckelbauteil 120 ausgebildet und kann im Spritzgießverfahren hergestellt werden. An einer Fuge 122 ist das Deckelbauteil 120 mit der Wandung 114 der Kammer 112 verschweißt. Der Einlaufverteiler 124 kann ebenfalls im Spritzgießverfaren ohne größeren Aufwand hergestellt werden und mittels der Leitschaufeln 128 mit der zweiten Leitfläche 134, die einen Teil der inneren Oberfläche der Kammer 112 bildet, durch Schweißen o.ä. verbunden werden. Weiterhin können Klemmverbindungen vorgesehen sein, so beispielsweise zwischen den Leitschaufeln und dem Tauchrohr. Zwischen den Oberkanten der Schaufeln 28 und der gegenüberliegenden zweiten Leitfläche kann dabei ein Spalt von etwa 0,2 mm verbleiben, um die Oberseite der Schaufeln bespülen zu können, wodurch unter anderem einer Konglomeration von Blutkörperchen entgegengewirkt wird.

Beide Ausführungsformen des erfindungsgemäßen Luftabscheiders werden vorzugsweise im Spritzgießverfahren aus durchsichtigem Kunststoff hergestellt, so daß eine optische Kontrolle des Füllstandes und des Strömungsverlaufes jederzeit einfach möglich ist.

Durch die erfindungsgemäße Ausgestaltung eines Luftabscheiders wird ein billig in großen Stückzahlen zu fertigender Luftabscheider geschaffen, der eine schnelle koaxiale Verbindung mit den Schläuchen eines Blutkreislaufes ermöglicht und nach Gebrauch entsorgt werden kann, wobei sortenreiner Kunststoffabfall anfällt.

Der Luftabscheider kann insbesondere auch bei allen medizinischen Flüssigkeiten zur Anwendung kommen.

Diese Flüssigkeiten können parenterale Lösungen, Plasma, Blut oder andere Blutbestandteile sein.

### Bezugszeichenliste

- 10, 110: Luftabscheider
- 12, 112: zylindrische Kammer
- 14, 114: Wandung (von 12, 112)
- 16, 116: Einlaufstutzen
- 18, 118: Auslaufstutzen
- 20, 120: Deckelbauteil
- 22, 122: Nahtfuge
- 24, 124: Strömungsleitbauteil (Einlaufverteiler)
- 26, 126: rotationssymmetrischer Grundkörper (von 24, 124)
- 28, 128: Leitschaufel
- 30: Einlauföffnung
- 32: Flansch
- 34, 134: zweite Leitfläche
- 36, 136: Strömungsleitkanal
- 38, 138: Luftpolster
- 40, 140: Flüssigkeitsspiegel
- 42: Filterkerze
- 119: Ablauföffnung
- 127: Kegelspitze, Staupunkt
- 132: kegelmantelförmiger Bereich (von 114)
- 144: Tauchrohr
- 145: Mündungsbereich (von 144)
- L: Längsachse

## Patentansprüche

1. Luftabscheider (10, 110) für Gasblasen enthaltende, durch eine Leitung geführte Flüssigkeit, insbesondere zum Abscheiden von Luftblasen aus Blut, mit einer im wesentlichen kreiszylinderförmigen Kammer (12, 112), die von der Flüssigkeit im wesentlichen auf schraubenlinienförmigen Strömungsbahnen durchflossen werden kann, so daß die Gasblasen wegen der durch die auftretenden Zentrifugalkräfte erzeugten Druckunterschiede in radialer Richtung zur Längsachse (L) der Kammer gedrängt werden können;
und mit einem Einlaufstutzen (16, 116) und einem Auslaufstutzen (18, 118),
**dadurch gekennzeichnet**, daß Einlaufstutzen und Ablaufstutzen in der Längsachse der Kammer liegend angeordnet sind,
daß stromab des Einlaufstutzens (16, 116) ein Strömungsleitbauteil (24 124) (Einlaufverteiler) angeordnet ist, das im wesentlichen aus einem rotationssymmetrischen Grundkörper (26, 126) besteht, dessen der einströmenden Flüssigkeit zugewandte äußere Oberfläche (erste Leitfläche) geometrisch durch Rotation eines Kurvenabschnitts um die Längsachse (L) der Kammer so definiert ist, daß die einströmende Flüssigkeit zunächst radial nach außen geleitet werden kann, und daß auf der Leitfläche Leitschaufeln (28, 128) angeordnet sind, die in auf der Längsachse senkrecht stehenden Ebenen gekrümmt und so umgelenkt sind, daß die Flüssigkeit beim Austritt aus dem Einlaufverteiler im wesentlichen tangential zur kreisförmigen Wandung (14) der Kammer strömen kann.

2. Luftabscheider nach Anspruch 1, **dadurch gekennzeichnet**, daß der die erste Leitfläche (26) erzeugende Kurvenabschnitt konkav gekrümmt ist.

3. Luftabscheider nach Anspruch 1, **dadurch gekennzeichnet**, daß der die erste Leitfläche (126) erzeugende Kurvenabschnitt ein Geradenabschnitt ist, so daß der Grundkörper des Strömungsleitbauteils kegelförmig ist.

4. Luftabscheider nach einem der vorhergehenden Ansprüche, **dadurch gekennzeichnet**, daß eine zweite Strömungsleitfläche (34, 134) mit Abstand parallel zur ersten Leitfläche angeordnet ist.

5. Luftabscheider nach Anspruch 4, **dadurch gekennzeichnet**, daß die Höhe der Leitschaufeln (28, 128) so bemessen ist, daß sie sich quer zur Strömungsrichtung von der ersten bis zur zweiten Leitfläche erstrecken.

6. Luftabscheider nach einem der vorhergehenden Ansprüche, insbesondere nach Anspruch 2, **dadurch gekennzeichnet**, daß die Kammer (12) im wesentlichen senkrecht angeordnet ist und für einen Durchfluß von oben nach unten ausgelegt ist.

7. Luftabscheider nach einem der Ansprüche 1 bis 5, insbesondere nach Anspruch 3, **dadurch gekennzeichnet**, daß die Kammer (112) im wesentlichen senkrecht angeordnet und für einen Durchfluß von unten nach oben ausgelegt ist.

8. Luftabscheider nach den Ansprüchen 3 und 7, **dadurch gekennzeichnet**, daß mit dem oben angeordneten Ablaufstutzen (118) ein Tauchrohr (144) verbunden ist, das bis in das Innere des kegelstumpfförmigen Strömungsleitbauteils (124) (Einlaufverteilers) reicht.

9. Luftabscheider nach einem der Ansprüche 7 oder 8 und nach Anspruch 4, **dadurch gekennzeichnet**, daß die Außenwand (114) der im wesentlichen kreiszylinderförmigen Kammer (114) einen anschließenden kegelförmigen Bereich (132) aufweist, dessen Innenseite die zweite Leitfläche (134) bildet.

10. Luftabscheider nach Anspruch 6, **dadurch gekennzeichnet**, daß die zweite Leitfläche (34) Teil der Oberfläche eines Flansches (32) ist, der mit einem Deckelbauteil (22) einstückig ausgebildet ist.

11. Luftabscheider nach Anspruch 2, **dadurch gekennzeichnet**, daß der erzeugende Kurvenabschnitt ein Ellipsenabschnitt ist.

12. Luftabscheider nach Anspruch 8, **dadurch gekennzeichnet**, daß das Tauchrohr (144) den Einlaufverteiler (124) sichert.

## Claims

1. An air separator (10, 110) for gas bubbles, containing fluid conducted through a pipe, in particular for the separation of air bubbles from blood, with a chamber (12, 112) essentially circular and cylindrical in shape, through which the liquid can flow in essentially helical flow paths, with the result that the gas bubbles can be pressed in a radial direction to the longitudinal axis (L) of the chamber as a result of the pressure differentials incurred by the centrifugal forces arising, and with an intake nozzle (16, 116) and an outlet nozzle (18, 118),
characterised in that the intake nozzle and outlet nozzle are arranged in the longitudinal axis of the chamber, that a flow guidance element (24, 124) is arranged downstream of the intake nozzle (16, 116), which consists essentially of a rotationally symmetrical basic body element (26, 126), the outer surface of which (the first deflection surface), turned towards the inflowing liquid, is defined geometrically by the rotation of a curve section about the longitudinal axis (L) of the chamber, in such a way that the inflowing liquid can initially be conducted radially outwards, and that vanes (28, 128) are arranged on the deflection surface which are curved in planes standing perpendicular to the longitudinal axis, and deflected in such a way that the liquid, on emerging from the influx distributor, flows essentially tangential to the circular walls of the chamber.

2. An air separator according to Claim 1, characterised in that the curved section forming the initial deflection surface (26) is curved in concave fashion.

3. An air separator according to Claim 1, characterised in that the curved section forming the first deflection surface (126) is a straight section, with the result that the basic body element of the flow guidance component is conical.

4. An air deflector according to one of the preceding Claims, characterised in that a second flow deflection surface (34, 134) is arranged at a distance from and parallel to the first deflection surface.

5. An air separator according to Claim 4, characterised in that the height of the vanes (28, 128) is dimensioned in such a way that they extend transversely to the direction of flow from the first to the second deflection surface.

6. An air separator according to one of the foregoing Claims, and Claim 2 in particular, characterised in that the chamber (12) is arranged essentially perpendicular, and is designed for a throughflow from top to bottom.

7. An air separator according to one of Claims 1 to 5, and Claim 3 in particular, characterised in that the chamber (112) is arranged essentially perpendicular, and is designed for a throughflow from bottom to top.

8. An air separator according to Claims 3 and 7, characterised in that an immersion tube (144) is connected to the outlet nozzle (118) arranged above, the said tube extending into the interior of the conically-shaped flow guidance element (124).

9. An air separator according to one of Claims 7 or 8, and in accordance with Claim 4, characterised in that the outer wall (114) of the essentially circular and cylindrical chamber (114) feature a connecting conical area (132), the inner face of which forms the second deflection surface (134).

10. An air separator according to Claim 6, characterised in that the second deflection surface (34) is a part of the surface of a flange (32), which is designed as one piece with a cover element (22).

11. An air separator according to Claim 2, characterised in that the curved section is designed as an elliptical section.

12. An air separator according to Claim 8, characterised in that the immersion tube (144) secures the influx distributor (124).

## Revendications

1. Séparateur d'air (10,110) pour un liquide contenant des bulles de gaz et véhiculé dans une canalisation, notamment pour séparer des bulles d'air du sang, comprenant une chambre essentiellement en forme de cylindre circulaire (12,112), qui peut être traversée par le liquide essentiellement suivant des trajectoires d'écoulement hélicoïdales, de sorte qu'en raison des différences de pression produites par les forces centrifuges qui apparaissent, les bulles de gaz peuvent être refoulées dans une direction radiale en direction de l'axe longitudinal (L) de la chambre, et
comportant un embout d'entrée (16,116) et un embout de sortie (18,118),
caractérisé en ce que l'embout d'entrée et l'embout de sortie sont disposés suivant l'axe longitudinal de la chambre, qu'en aval de l'embout d'entrée (16,116) est disposé un composant (24,124) de guidage de l'écoulement (répartiteur d'entrée), qui est constitué essentiellement par un corps de base à symétrie de révolution (26,126), dont la surface extérieure (première surface de guidage), qui est tournée vers le liquide entrant, est définie géométriquement par rotation d'une section de courbe autour de l'axe longitudinal (L) de la chambre de telle sorte que le liquide entrant peut être tout d'abord dirigé radialement vers l'extérieur, que sur la surface de guidage sont disposées des aubes directrices (28,128), qui sont cintrées dans des plans perpendiculaires à l'axe longitudinal et sont courbées de telle sorte que, lorsque le liquide sort du répartiteur d'entrée, il peut circuler essentiellement tangentiellement en direction de la paroi circulaire (14) de la chambre.

2. Séparateur d'air selon la revendication 1, caractérisé en ce que la section de courbe, qui produit la première surface de guidage (26), est cintrée avec une forme concave.

3. Séparateur d'air selon la revendication 1, caractérisé en ce que la section de courbe, qui produit la première surface de guidage (126), est un segment de droite de sorte que le corps de base du composant de guidage de l'écoulement a une forme conique.

4. Séparateur d'air selon l'une des revendications précédentes, caractérisé en ce qu'une seconde surface (34,134) de guidage de l'écoulement est disposée à distance et parallèlement à la première surface de guidage.

5. Séparateur d'air selon la revendication 4, caractérisé en ce que la hauteur des aubes directrices (28,128) est dimensionnée de telle sorte qu'elle s'étend transversalement par rapport à la direction d'écoulement de la première jusqu'à la seconde surface de guidage.

6. Séparateur d'air selon l'une des revendications précédentes, notamment la revendication 2, caractérisé en ce que la chambre (12) est disposée essentiellement verticalement et est agencée pour une circulation descendante.

7. Séparateur d'air selon l'une des revendications 1 à 5, notamment selon la revendication 3, caractérisé en ce que la chambre (112) est disposée de manière à être essentiellement verticale et est conçue pour une circulation ascendante.

8. Séparateur d'air selon les revendications 3 et 7, caractérisé en ce qu'à l'embout de sortie (118) disposé à la partie supérieure est raccordé un tube plongeur (144) qui s'étend jusqu'à l'intérieur du composant de forme tronconique (124) de guidage de l'écoulement (répartiteur d'entrée).

9. Séparateur d'air selon l'une des revendications 7 ou 8 et selon la revendication 4, caractérisé en ce que la paroi extérieure (114) de la chambre essentiellement en forme de cylindre circulaire (112) comporte une partie contiguë de forme conique (132), dont la face intérieure forme la seconde surface de guidage (134).

10. Séparateur d'air selon la revendication 6, caractérisé en ce que la seconde surface de guidage (34) fait partie de la surface d'une bride (32), qui est agencé d'un seul tenant avec un composant formant couvercle (22).

11. Séparateur d'air selon la revendication 2, caractérisé en ce que la section de courbe génératrice est une section d'ellipse.

12. Séparateur d'air selon la revendication 8, caractérisé en ce que le tube de plongée (144) fixe le répartiteur d'entrée (124).
